# EUROPÄISCHE PATENTANMELDUNG

(11) **EP 0 644 503 A1**
(43) Veröffentlichungstag der Anmeldung: **22.03.1995**
(21) Anmeldenummer: 94112613.8
(22) Anmeldetag: 12.08.1994
(51) Int. Cl.: G06K 7/10

(54) **Lesevorrichtung für am Umfang von Flaschen für Milchproben angebrachte Barcodes**

(30) Priorität: 21.09.1993 DE 4331871
(71) Anmelder: JANSKY GMBH, D-48282 Emsdetten (DE)
(72) Erfinder: Jansky, Dipl. Ing. Manfred, D-48282 Emsdetten (DE)
(74) Vertreter: Cohausz & Florack

(57) **Zusammenfassung**

Um am Umfang von in einem Stativ (3) in Reihe hintereinander angeordneten Probeflaschen (4) angebrachte Barcodes (5) lesen zu können, wird das Stativ (3) mit den Probeflaschen (4) schrittweise über eine Führungsbahn (8) an einem stationär angeordneten Lesekopf (20) vorbeibewegt. Nach jedem Schritt wird die Flasche (4) um ihre eigene Achse gedreht, so daß der Lesekopf den Barcode (5) lesen kann. Die Drehbewegung erfolgt mittels einer magnetischen, angetriebenen Drehscheibe (17), die in einem Schieber (12) gelagert ist, der von der Seite unter den aus ferromagnetischem Material bestehenden Boden (6) der Flasche (4) schiebbar ist.

## Beschreibung

Beim Einsammeln von Milch bei verschiedenen Lieferanten mittels eines Milchsammelwagens ist es erforderlich, von den einzelnen bei den Lieferanten angenommenen Milchvolumina repräsentative Milchproben zu entnehmen. Das Abfüllen solcher Milchproben geschieht automatisch in Probeflaschen, die mit einem Barcode versehen sind und in Reihe in einem Rundstativ stehen. Ein auf dem Milchsammelwagen installierter Rechner hält fest, welche Probeflasche bei welchem Lieferanten mit Milch gefüllt wurde. Dafür ist es erforderlich, daß eine Lesevorrichtung vorgesehen ist, die den Barcode an der jeweiligen Flasche liest, und daß der Rechner diesen Barcode dem Lieferanten zuordnet. Mit diesen gespeicherten Daten ist es grundsätzlich möglich, bei der späteren Untersuchung der Milchprobe in einem Labor diese den einzelnen Lieferanten wieder zuzuordnen. Diese Untersuchung ist nicht an die Reihenfolge der Probeflaschen im Rundstativ gebunden.

In der Regel erfolgt die Untersuchung in Laborgeräten, die für die Untersuchung der Milchproben nicht in einem Rundstativ, sondern in einem Längsstativ ausgelegt sind. Deshalb ist es erforderlich, die Probeflaschen aus den Rundstativen des Milchsammelwagens in die für die Laborgeräte geeigneten Längsstative umzuladen. Da diese Laborgeräte nicht mit Barcodes arbeiten, sondern das Untersuchungsergebnis dem Platz im Stativ zuordnen, kommt es für eine einwandfreie Zuordnung der Probe zum Lieferanten darauf an, daß die Reihenfolge der Probeflaschen im Rundstativ des Milchsammelwagens und im Längsstativ des Laborgerätes beibehalten wird.

Aus der deutschen Offenlegungsschrift DE 41 35 420 A1 ist ein Probenflaschen- Rundmagazin bekannt, bei dem das Umfallen von gefüllten Probenflaschen zuverlässig verhindert wird. Zu diesem Zweck weist das bekannte Probenflaschen-Rundmagazin einen mit einem Reversier-Antrieb versehenen magnetischen Drehboden mit einer darüber ortsfest angeordneten Spirale auf, der eine beidseitige seitliche Abstützung für die einreihig durch den Drehboden transportierenden Probeflaschen bildet. Die Probeflaschen treten aus einer Probeflaschen-Übergabe- und -Einspeisevorrichtung in die Spirale des Rundmagazins ein. Anschließend wird der magnetische Drehboden entgegen der Eintrittsrichtung der Probeflaschen derart bewegt, daß die schon in der Spirale des Rundmagazins sich befindenden Probenflaschen in dichter Anlage aneinander geschoben werden. Anschließend wird die Drehrichtung des Drehbodens umgekehrt, so daß die nun dicht aneinander stehenden Probenflaschen als zusammenhängende Einheit in der Spirale des Rundmagazins weitergefördert werden.

Probenflaschen, die beispielsweise zur Verarbeitung in einem Rundmagazin der vorstehend genannten Art geeignet sind, sind aus der deutschen Offenlegungsschrift DE 39 25 165 A1 bekannt. Diese Rundflaschen weisen ein topfförmiges Ausrichtelement aus magnetisierbarem Material auf, welches die Standfläche des eigentlichen Probenflaschenkörpers bilden.

Eine weitere Probenflaschen-Transportvorrichtung ist aus der deutschen Patentschrift DE 37 05 813 C1 bekannt. Diese bekannte Transportvorrichtung dient als Verbindung zwischen zwei Kassetten, in denen Probenflaschen auf einem Milchsammelwagen transportiert werden können. Die bekannte Transportvorrichtung besteht aus einer Transportbrücke und einer Kulissenführung, über die die Probenflaschen auf direktem Weg von der einen Kassette zu der anderen Kassette bewegt werden können. Dabei ist auf dem Transportweg eine vorbestimmte Halteposition vorgesehen, in der die Flaschen mittels eines Drehtellers für die Auslesung eines Barcodes gedreht werden können, der auf der Probenflasche befestigt ist.

Die Erfahrung hat gezeigt, daß es beim Umladen von Probeflaschen immer wieder zu Verwechslungen kommt.

Der Erfindung liegt die Aufgabe Zugrunde, eine Lesevorrichtung für am Umfang von Flaschen für Milchproben angebrachte Barcodes zu schaffen, die das Lesen der Barcodes von in Stativen eines Laborgerätes stehenden Flaschen, insbesondere in Längsstativen stehenden Flaschen, ermöglicht.

Diese Aufgabe wird mit einer Lesevorrichtung für am Umfang von Flaschen für Milchproben angebrachte Barcodes mit einem Stativ, in dem die Flaschen in Reihe hintereinander und drehbeweglich angeordnet sind, und mit einem am Stativ angreifenden Transporteur, der die Flaschen nacheinander an einem stationär angeordneten Lesekopf für die Barcodes vorbeibewegt, gelöst, wobei die mit einem Boden aus ferromagnetischem Material ausgestatteten Flaschen axial beweglich im Stativ gehalten sind und am Ort des Lesekopfes von der Seite aus eine von einem Schieber getragene angetriebene Drehscheibe aus magnetischem Material unter den Boden schiebbar ist.

Bei der Erfindung wird eine an sich bekannte, mit einem ferromagnetischen Boden ausgestattete Flasche verwendet, um den Boden als Teil einer aus dem Boden und der Drehscheibe gebildeten kraftschlüssigen Kupplung zur Übertragung eines Drehmomentes zu nutzen. Ohne großen Aufwand läßt sich deshalb auf die Flasche ein Drehmoment ausüben, so daß die im Stativ stehende Flasche in eine für das Lesen des Barcodes optimale Lage dem Leserkopf präsentiert wird.

Für den Antrieb der Drehscheibe bieten sich verschiedene Möglichkeiten. Nach einer besonders einfach ausgestalteten Lösung ist die Drehscheibe über einen Riemen angetrieben.

Um den Schieber leicht unter den Boden der Flasche schieben zu können, kann er nach einer Ausgestaltung der Erfindung an seinem der Flasche zugewandten Ende eine keilförmige Rampe aufweisen.

Das Stativ kann als quaderförmiger länglicher Kasten ausgebildet sein. In diesem Fall umfaßt der Transporteur eine Führungsbahn, die auf der dem Schieber abgewandten Seite eine Stützschiene für das Stativ aufweist, wobei ein federnd nachgiebiges, gemeinsam mit dem Schieber gegen das Stativ vorsteuerbares Positionierelement vorgesehen ist, das das Stativ gegen die Stützschiene drückt. Diese Ausgestaltung der Erfindung garantiert, daß im Augenblick des Lesens des Barcodes das Stativ und damit auch die in ihm gehaltenen Probeflaschen in einer definierten Position gegenüber dem Lesekopf gehalten werden.

Zur Verbesserung des Haltens der Flaschen im Stativ beim Einschieben des Schiebers sollte das Stativ auf gegenüberliegenden Seiten axial versetzte Stützschienen aufweisen, von denen die auf der dem Schieber abgewandten Seite angeordnete Stützschiene dem Boden der Flaschen näher als die andere Schiene liegt. Durch diese Anordnung erreicht man, daß beim Einschieben des Schiebers die Flaschen nicht kippen, die Flaschen aber drehbeweglich bleiben und nicht verkanten.

Vorzugsweise sind in der dem Schieber zugewandten Seite des Stativs Fenster zum Einführen des Schiebers vorgesehen. Um dessen Einführen zu erleichtern, kann der Boden des Stativs im Bereich der Fenster als schräge Auflauframpe ausgebildet sein.

Für einen schrittweisen Vorschub des Stativs entsprechend dem Abstand der Flaschen in der Reihe weist der Transporteur ein Schrittschaltwerk auf, für das am Stativ Angriffsnocken oder Ausnehmungen vorgesehen sind.

Im folgenden wird die Erfindung anhand einer ein Ausführungsbeispiel darstellenden Zeichnung näher erläutert. Im einzelnen zeigen
- Fig. 1: die Lesevorrichtung mit Stativ im Querschnitt während des Vorschubes des Stativs,
- Fig. 2: die Lesevorrichtung gemäß Fig. 1 im Querschnitt während des Lesens eines Barcodes,
- Fig. 3: einen Teil eines leeren Stativs in perspektivischer Darstellung,
- Fig. 4: das Stativ der Fig. 3 in Seitenansicht aus Richtung des Pfeiles A der Fig. 3,
- Fig. 5: das Stativ gemäß Fig. 3 im Querschnitt und
- Fig. 6: das Stativ gemäß Fig. 3 in Seitenansicht aus der Richtung des Pfeiles B.

Auf einem Unterbau 1 mit oberseitig angeordneter Führungsbahn 2 befindet sich ein kastenförmiges Stativ 3 mit darin in Reihe hintereinander angeordneten Probeflaschen (Flaschen) 4 für Milch. Die Probeflaschen 4 tragen an ihrem Umfang einen Barcode 5 und haben einen topfförmigen Boden 6 aus ferromagnetischem Material. Die Führungsbahn 2 weist seitliche Führungs- und Stützschienen 7, 8 für das Stativ 3 auf. An einer Seite des Unterbaues 1 ist ein Drehantrieb 9 angebracht. Der Drehantrieb 9 besteht aus einem auf Schienen 10 geführten Schlitten 11 mit einem von ihm getragenen Schieber 12 und einem ebenfalls vom Schlitten 1 getragenen Antriebsmotor 13. Der Schlitten 11 ist mittels eines Zylinderkolbenantriebs 14 auf den Schienen 10 verfahrbar.

Der Schieber 12 weist an seinem vorderen Ende eine keilförmige Rampe 16 auf. Auf seiner Oberseite trägt er eine drehbar gelagerte Drehscheibe 17, die aus magnetischem Material besteht und über einen Riemen 18 vom Antriebsmotor 13 angetrieben ist. Zur Führung der Drehscheibe 17 und der beiden Stränge des Riemens 18 ist auf dem Schieber ein Führungselement 19 angeordnet, das mit der Drehscheibe 17 eine gemeinsame obere Ebene bildet.

Am Unterbau 1 seitlich des Transportweges des Stativs 13 ist ein Lesekopf 20 für die Barcodes 5 der Flaschen 4 stationär angebaut.

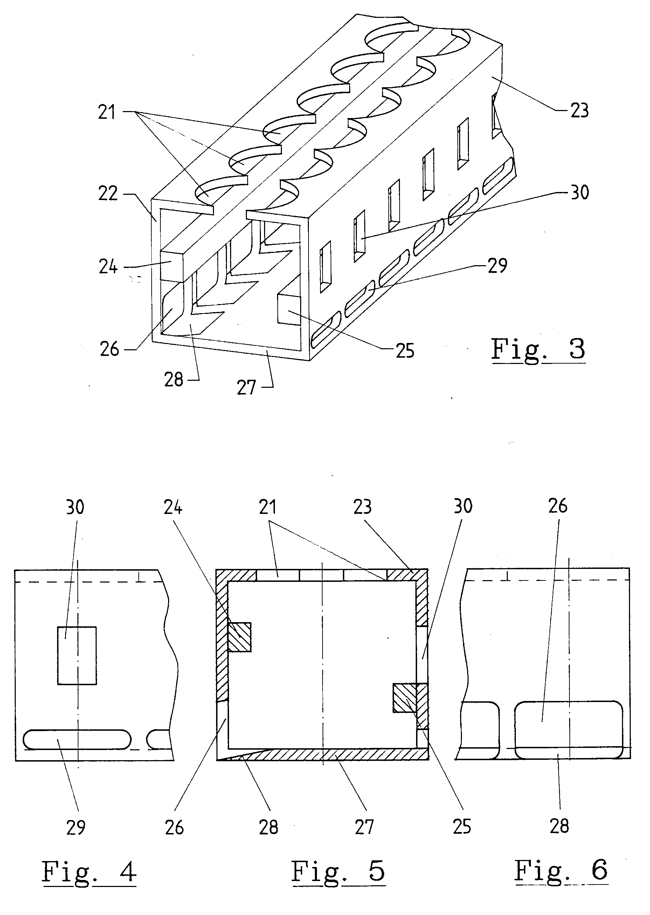

Das Stativ 3, das im einzelnen in den Fig. 3 bis 6 dargestellt ist, hat die Grundform eines länglichen quaderförmigen Kastens. In der Oberseite weist es mehrere Aufnahmeplätze für die Flaschen 4 auf, die jeweils von einem Paar kreisbogenförmiger Ausnehmungen in der Oberseite des Stativs 3 gebildet sind. An den Innenseiten der Seitenwände 22, 23 sind Stützschienen 24, 25 höhenmäßig gegeneinander versetzt für die Flaschen 4 vorgesehen. Unterhalb der dem Schieber 12 zugewandten Stützschiene 24 sind Fenster 26 vorgesehen, in deren Bereich der Boden eine schräge Rampe 28 aufweist. Durch diese Fenster 26 läßt sich der Schieber 12 in das Stativ 3 unter den Boden 6 der Flaschen 4 einschieben, wobei das Einschieben durch die Auflauframpe 28 erleichtert wird. Auf der gegenüberliegenden Seite des Fensters 26 ist ein weiteres Fenster 29 vorgesehen, das in der Höhe allerdings kleiner ist. Es dient dazu, bei eingeschobenem Schieber 12 die Spitze seiner keilförmigen Rampe 16 aufzunehmen. Oberhalb dieser Fenster 29 sind Ausnehmungen 30 vorgesehen, in die ein Greifer eines nicht dargestellten Schrittschaltwerkes eingreift, das das Stativ 3 verschiebt.

Der Antriebsmotor 13 trägt auf seiner dem Stativ 3 zugewandten Seite eine Feder 31, die das Stativ 3 bei vorgesteuertem Schieber 12 gegen die Führungs- und Stützschiene 8 drückt.

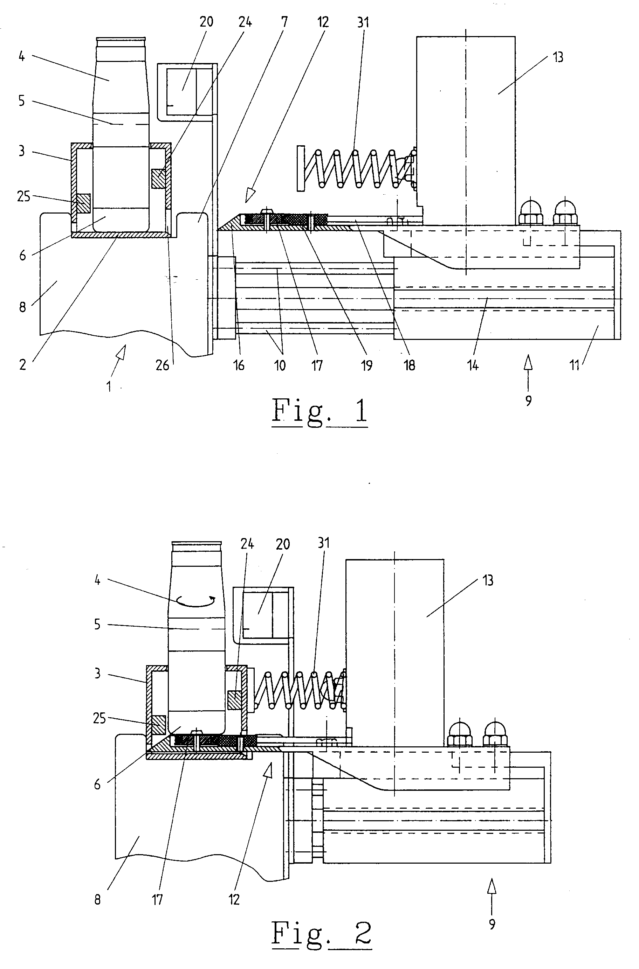

Die beschriebene Lesevorrichtung arbeitet auf folgende Art und Weise:
Nachdem ein mit Probeflaschen 4 gefülltes Stativ 3 auf die Führungsbahn 2 gesetzt worden ist, wird es von dem nicht dargestellten Schrittschaltwerk schrittweise vorbewegt, und zwar derart, daß das Stativ 3 stillsteht, wenn eine Flasche 4 dem Lesekopf 20 gegenüberliegt. Von der Seite wird dann durch den Zylinderkolbenantrieb 14 der Schieber 12 durch das Fenster 26 unter die Flasche 4 geschoben, bis daß die Drehscheibe 17 zentral unter der Flasche 4 sich befindet, wie in Fig. 2 dargestellt ist. Dabei wird die Flasche 4 angehoben, so daß der Barcode 5 dem Eingang des Lesekopfes 20 genau gegenüberliegt. Durch Magnetkraft ist die Flasche 4 mit ihrem aus ferromagnetischem Material bestehenden topfförmigen Boden 6 mit der aus magnetischem Material bestehenden Drehscheibe drehfest gekuppelt. Beim Einschieben des Schiebers 12 stützt sich die Flasche 4 einerseits an der unteren Schiene 25 und andererseits an der oberen Schiene 24 ab. Dadurch wird ein Kippen verhindert, so daß sie wackelfrei aber drehbeweglich gehalten ist. Das Stativ 3 selbst wird durch die Feder 31 in Anlage an die Stützschiene 8 gehalten, so daß die Flasche 4 sich in einer für das Auslesen des Barcodes 5 optimalen Position befindet. Durch den Antriebsmotor 13 wird über den Antriebsriemen 18 die mit ihrem Boden 6 an die Drehscheibe 17 gekuppelte Flasche 4 in Drehung versetzt, so daß der Barcode 5 vom Lesekopf 20 gelesen werden kann. Nach diesem Lesevorgang wird der Schieber 12 zurückbewegt und das Stativ 3 um einen Schritt durch das Schrittschaltwerk weitertransportiert. Der Vorgang des Anhebens und Ankuppelns der Flasche 4 an die Drehscheibe 17 wiederholt sich dann.

## Patentansprüche

1. Lesevorrichtung für am Umfang von Flaschen (4) für Milchproben angebrachte Barcodes (5) mit einem Stativ (3), in dem die Flaschen (4) in Reihe hintereinander und drehbeweglich angeordnet sind und mit einem am Stativ (3) angreifenden Transporteur, der die Flaschen (4) nacheinander an einem stationär angeordneten Lesekopf (20) für die Barcodes (5) vorbeibewegt, wobei die mit einem Boden (6) aus ferromagnetischen Material ausgestatteten Flaschen (4) axial beweglich im Stativ (3) gehalten sind, und wobei am Ort des Lesekopfes (20) von der Seite aus eine von einem Schieber (12) getragene, angetriebene Drehscheibe (17) aus magnetischem Material unter den Boden (6) schiebbar ist.

2. Lesevorrichtung nach Anspruch 1, dadurch gekennzeichnet, daß die Drehscheibe (17) über einen Riemen (18) angetrieben ist.

3. Lesevorrichtung nach Anspruch 1 oder 2, dadurch gekennzeichnet, daß der Schieber (12) an seinem der Flasche (4) zugewandten Ende eine keilförmige Rampe (16) aufweist.

4. Lesevorrichtung nach einem der Ansprüche 1 bis 3, dadurch gekennzeichnet, daß der Transporteur eine Führungsbahn (2) für das kastenförmige Stativ (3) umfaßt, die auf der dem Schieber (12) abgewandten Seite eine Stützschiene (8) für das Stativ (3) aufweist, und daß eine federnd nachgiebiges, gemeinsam mit dem Schieber (12) gegen das Stativ (3) vorsteuerbares Positionierelement (31) vorgesehen ist, das das Stativ (3) gegen die Stützschiene (8) drückt.

5. Lesevorrichtung nach einem der Ansprüche 1 bis 4, dadurch gekennzeichnet, daß das Stativ (3) für die Flaschen (4) auf gegenüberliegenden Seiten axial versetzte Stützschienen (24, 25) aufweist, von denen die auf der dem Schieber (12) abgewandten Seite angeordnete Schiene (25) näher dem Boden (6) der Flasche (4) als die andere Schiene (24) liegt.

6. Lesevorrichtung nach einem der Ansprüche 1 bis 5, dadurch gekennzeichnet, daß in der dem Schieber (12) zugewandten Seite (22) des Stativs (3) Fenster (26) zum Einführen des Schiebers (12) vorgesehen sind.

7. Lesevorrichtung nach Anspruch 6, dadurch gekennzeichnet, daß der Boden (27) des Stativs (3) im Bereich der Fenster (26) als schräge Auflauframpe (28) für den Schieber (12) ausgebildet ist.

8. Lesevorrichtung nach einem der Ansprüche 1 bis 7, dadurch gekennzeichnet, daß der Transporteur ein Schrittschaltwerk für den Vorschub des Stativs (3) aufweist, für das am Stativ (3) Angriffsnocken oder Ausnehmungen (30) vorgesehen sind.
